# EUROPEAN PATENT SPECIFICATION

(11) **EP 3 984 486 B1**
(45) Date of publication and mention of the grant of the patent: **07.10.2026**
(21) Application number: 19935877.1
(22) Date of filing: 28.10.2019
(51) Int. Cl.: A61B 34/20, A61B 90/00, G06T 19/00, A61B 3/10, G01B 9/02015, G01B 9/02091

(54) **OPTICAL COHERENCE TOMOGRAPHY AUGMENTED REALITY-BASED SURGICAL MICROSCOPE IMAGING SYSTEM AND METHOD**
BILDGEBUNGSSYSTEM UND -VERFAHREN FÜR CHIRURGISCHES MIKROSKOP AUF BASIS VON ERWEITERTER REALITÄT MIT OPTISCHER KOHÄRENZTOMOGRAFIE
SYSTÈME ET PROCÉDÉ D'IMAGERIE PAR MICROSCOPE CHIRURGICAL BASÉE SUR LA RÉALITÉ AUGMENTÉE PAR TOMOGRAPHIE PAR COHÉRENCE OPTIQUE

(30) Priority: 01.07.2019 CN 201910583463
(43) Date of publication of application: 20.04.2022
(73) Proprietor: Suzhou Institute of Biomedical Engineering and Technology Chinese Academy of Sciences, Suzhou, Jiangsu 215163 (CN)
(72) Inventor: SHI, Guohua, Suzhou, Jiangsu 215163 (CN); FAN, Jinyu, Xiangtan, Hunan 411207 (CN); HE, Yi, Chengdu, Sichuan 610041 (CN); XIN, Lina, Changchun, Jilin 130033 (CN); GAO, Feng, Jiangxi 336000 (CN)
(74) Representative: Groth & Co. KB
(86) International application number: PCT/CN2019/113695
(87) International publication number: WO 2021/000466

(56) References cited:
- WO-A1-2016/172495
- CN-A- 103 118 585
- CN-A- 103 892 919
- CN-A- 107 920 918
- CN-A- 108 289 607
- CN-A- 108 577 802
- US-A- 6 004 314
- US-A1- 2010 302 508
- US-A1- 2015 342 460
- US-A1- 2017 156 588
- US-A1- 2017 280 989
- US-A1- 2019 175 402

## Description

### TECHNICAL FIELD

The disclosure relates to the technical field of microsurgery imaging and graphic processing, and particularly relates to an Optical Coherence Tomography (OCT) augmented reality-based surgical microscope imaging system.

### BACKGROUD

Modem surgery has required that when a surgical target site is positioned, the physiological trauma on patients should be reduced to the greatest extent to achieve minimally invasive surgery. Image-guided interventional surgery can accurately position the surgical target site, can achieve the characteristics of preoperative planning, intra-operative real-time monitoring navigation, postoperative evaluation on the surgery effect of a surgical region and the like, has the advantages of high accuracy, small trauma and the like, and is an important direction of the modern surgery.

Currently, imaging ranges of optical microscope-based microsurgery such as ophthalmic surgery and neurosurgery are limited to surface two-dimensional imaging, resulting in severe limitation to application of the microsurgery. OCT is a high-resolution high-sensitivity non-contact three-dimensional imaging method, can be used for carrying out imaging on tomography inside tissues and surgical instruments, and is particularly applicable to navigation of fine surgery, so that a Microscope Integrated OCT (MIOCT) surgical navigation system is developed, and meanwhile, development of a high-Speed Sweep-frequency OCT (SS-OCT) technology enables intra-operative application of three-dimensional OCT real-time imaging to become possible. The patent application US2017/280989A1 relates generally to surgical procedures, and more particularly to ophthalmic surgical visualization systems and methods. The patent WO2016/172495A1 provides a MIOCT imaging display method in which OCT information and microscope information are simultaneously displayed in an eyepiece. However, in the method, an OCT image is just displayed beside a microscopic image, fused imaging of the OCT image and the microscopic image is not involved, and thus a doctor still needs to carry out subjective matching on the two images during surgery. In order to solve the above problem, the surgical imaging method and equipment need to be improved to acquire more intuitive intra-operative navigation information.

Augmented reality is a technology of fusing the scene in a virtual world on display equipment with the scene in a real world through position and angle refined calculation of a camera video and an image analysis technology. In surgery, the augmented reality technology can fuse a three-dimensional (3D) image such as CT with a real scene so as to implement intuitive surgical guidance. The key of the augmented reality technology is virtual-real registration, i.e., establishment of a coordinate transformation relationship of a virtual image and the real scene, and the difficulty thereof lies in finding positions of the same point in virtual and real coordinate systems, i.e., setting and tracking a datum point. When an artificially placed object is used as the datum point for registration, a matching result with better accuracy can be obtained, but the method possibly causes traumas; and when body surface characteristics are utilized to set the datum point, the additional traumas can be avoided, but when the characteristics are unobvious, the identification effect is poor, resulting in limitation to the application of the method. Therefore, in order to fuse a three-dimensional OCT image serving as a virtual image into the microscopic image, new registration and fusion method and imaging system need to be introduced.

### SUMMARY

The present disclosure is to solve the technical problem of providing an OCT augmented reality-based surgical microscope imaging system and method for the above-mentioned defects in the prior art. The invention is defined by claims 1 and 6.

In order to solve the above-mentioned technical problem, the present disclosure adopts the technical solution that an OCT augmented reality-based surgical microscope imaging system includes:
a surgical microscope unit, configured to acquire a two-dimensional microscopic image of a surgical region;
an OCT unit, configured to acquire an OCT three-dimensional image of the surgical region;
a processing control unit, configured to acquire the two-dimensional microscopic image and the OCT three-dimensional image of the surgical region, and an image obtained by fusing the two-dimensional microscopic image and the OCT three-dimensional image of the surgical region; and
a display unit, configured to output and display a result of the processing control unit to carry out navigation for surgery.

The system further includes a guidance light source, which can be captured by the surgical microscope unit and is configured to project, into the surgical region, a guidance light spot synchronized with an OCT scanning light source of the OCT unit.

Preferably, the system further includes a surgical lighting unit and an objective lens, a light splitting unit and an optical zoom unit sequentially arranged along an imaging optical path of the surgical microscope unit;
the surgical lighting unit is configured to provide lighting light for the surgical region, and the lighting light reflected by the surgical region enters the surgical microscope unit after sequentially passing through the objective lens, the light splitting unit and the optical zoom unit so as to implement two-dimensional microscopic imaging of the surgical region; and
light emitted by the guidance light source and the OCT scanning light source of the OCT unit reaches the surgical region after sequentially passing through the light splitting unit and the objective lens, and OCT scanning light reflected by the surgical region backtracks to the OCT unit to implement OCT three-dimensional imaging; and after guidance light reflected by the surgical region passes through the light splitting unit, one portion of the guidance light enters the OCT unit, while the other portion of the guidance light enters the surgical microscope unit.

Preferably, the surgical microscope unit includes imaging lenses and cameras, the imaging lenses include a left imaging lens and a right imaging lens, and the cameras include a left camera and a right camera, wherein the left imaging lens and the left camera correspondingly constitute a left microscopic imaging module, and the right imaging lens and the right camera correspondingly constitute a right microscopic imaging module.

Preferably, the light splitting unit is a dichroic mirror which carries out total reflection on the light of the OCT unit, carries out semi-transmission and semi-reflection on the light of the guidance light source, and carries out total transmission on the light of the surgical lighting unit.

Preferably, the OCT unit includes the OCT scanning light source, a first coupler, a wavelength division multiplexer, a first collimator, a two-dimensional galvanometer scanner, a second collimator, a reflector, a third collimator, a second coupler and a balance detector;
an OCT scanning beam emitted by the OCT scanning light source is split into two paths of light via the first coupler, one path of light is sample light, and the other path of light is reference light;
the guidance light emitted by the guidance light source and the sample light are converged via the wavelength division multiplexer, then pass through the first collimator together to become incident to the two-dimensional galvanometer scanner to be deflected, and then are focused into the surgical region by the objective lens after being reflected by the dichroic mirror;
both the sample light and one portion of guidance light reflected by the surgical region return along an original path after being reflected by the dichroic mirror, and reach one end of the second coupler after passing through the first coupler; the other portion of guidance light reflected by the surgical region transmits through the dichroic mirror after passing through the objective lens, passes through the optical zoom unit, and then respectively passes through the left imaging lens and the right imaging lens to respectively enter the left camera and the right camera;
the reference light emergent after passing through the first coupler sequentially passes through the second collimator, the reflector and the third collimator to reach said one end of the second coupler, and enters the second coupler together with the sample light and said one portion of guidance light that have been reflected by the surgical region and reached said one end of the second coupler, the reference light undergoes interference with the sample light and said one portion of guidance light before being received by the balance detector, and finally, a detection result is output to the processing control unit so as to implement OCT three-dimensional imaging;
after a lighting beam emitted by the surgical lighting unit irradiates the surgical region, the lighting light and the other portion of guidance light reflected by the surgical region transmit through the dichroic mirror, then pass through the optical zoom unit, subsequently enter the left microscopic imaging module and the right microscopic imaging module, and finally, an image signal is output to the processing control unit so as to implement two-dimensional microscopic imaging of the surgical region; and
the processing control unit carries out registration and fusion on the two-dimensional microscopic image and the OCT three-dimensional image of the surgical region, and a fused image is displayed and output by the display unit so as to carry out navigation for surgery.

Preferably, the display unit is a polarized light display screen with a stereoscopic visual effect, and is configured to respectively output an image obtained by fusing the two-dimensional microscopic image from the left microscopic imaging module and the OCT three-dimensional image and output an image obtained by fusing the two-dimensional microscopic image from the right microscopic imaging module and the OCT three-dimensional image.

An OCT augmented reality-based surgical microscope imaging method uses the system as mentioned above to carry out imaging, and includes the following steps:
S1: adjusting the output intensity and focus positions of a surgical lighting unit and a guidance light source to enable cameras of a surgical microscope unit to clearly observe a surgical region and a guidance light spot, and acquiring a microscopic image of the surgical region;
S2: establishing a microscope two-dimensional Cartesian coordinate system *Ox₀y₀* by taking a two-dimensional plane of the microscopic image acquired by the cameras as *x* and *y* axes, obtaining coordinates of the guidance light spot in the microscope coordinate system according to a position of the guidance light spot in the image, and using the obtained coordinates as a datum point; and changing, in an OCT three-dimensional scanning region, a deflection angle of a two-dimensional galvanometer scanner, acquiring coordinates of a series of different datum points to be marked as {A₁, A₂ ... Aₙ};
S3: establishing a three-dimensional Cartesian coordinate system *Ox₀y₀z₀,* named an OCT coordinate system, by taking a plurality of pieces of continuous OCT slicing data at adjacent positions as volume data, taking an OCT depth scanning direction as a z axis and taking scanning directions of the two-dimensional galvanometer scanner as x and y axes; carrying out primary OCT three-dimensional scanning on an imaging region, wherein due to the fact that a scanner deflection angle corresponding to the projection position of guidance light in the step S2 is known, coordinate values of *x₁* and *y₁,* corresponding to the position of the guidance light spot of the step S2, in the OCT coordinate system is also known, finding a boundary where the guidance light spot is located according to an OCT structure, thus acquiring coordinate values of z₁ of the guidance light spot of the step S2 in the OCT coordinate system, and finally, obtaining coordinates {*B₁, B₂* ... *Bₙ*} in the OCT coordinate system corresponding to the datum points {*A₁,* A₂ ... *Aₙ*} in the microscope two-dimensional Cartesian coordinate system *Ox₀y₀*;
S4: carrying out fitting on {*A₁, A₂* ... *Aₙ*} and {*B₁, B₂* ... *Bₙ*} to obtain a transformation relationship from the OCT coordinate system to the microscope two-dimensional Cartesian coordinate system, which is a homography matrix corresponding to coordinate transformation, calibrating the cameras to obtain internal parameters of the cameras, and carrying out matrix operation to obtain external parameters of the cameras;
S5: adjusting the intensity of the surgical lighting unit, and simultaneously starting to carry out OCT three-dimensional scanning on the surgical region;
S6: setting virtual camera parameters of an OCT three-dimensional reconstructed portion according to the microscope external parameters obtained in the step S4 so as to obtain a registered OCT three-dimensional reconstructed image, and finally, carrying out superposition on the registered OCT three-dimensional reconstructed image and the microscopic image of the surgical region to complete virtual-and-real-image fusion display; and
S7: repeating the step S6 as OCT scanning continuously updates the input volume data, reconstructing all two-dimensional structural images to form a three-dimensional tomography model of the surgical region, and carrying out display by a display unit so as to implement real-time augmentation on the microscopic image of the surgical region.

Preferably, corresponding to the left camera and the right camera, respective microscope coordinate systems need to be respectively established, and then registration and fusion are carried out with an OCT image respectively.

Preferably, when the position of the datum point is set, the defection angle of the two-dimensional galvanometer scanner is a value during OCT three-dimensional scanning, instead of a random value in a scannable range.

Preferably, a number of the datum points required in the step S2 is n, n≥6.

The present disclosure has the beneficial effect that the surgical microscopic imaging system and method can accurately carry out registration and fusion on the two-dimensional microscopic image and the OCT three-dimensional image, thereby implementing real-time enhancement on the microscopic image of the surgical region, providing more intuitive navigation information for surgery, and implementing intuitive surgical guidance.

### BRIEF DESCRIPTION OF THE DRAWINGS

Fig.1 is a schematic block diagram of configuration of an imaging system in accordance with an embodiment of the present disclosure;
Fig. 2 is a detail view of a structure of an imaging system in accordance with an embodiment of the present disclosure;
Fig. 3 is a flow chart of image fusion in accordance with an embodiment of the present disclosure;
Fig. 4 is a schematic diagram of establishment of a coordinate system and setting and search of a datum point in accordance with an embodiment of the present disclosure;
Fig. 5 shows a process and a result of fusion of a finger microscopic image and an OCT image in accordance with an embodiment of the present disclosure; and
Fig. 6 is a schematic diagram showing a spatial relationship of each coordinate system in accordance with an embodiment of the present disclosure.

### Description of reference numerals:

1-surgical region; 2-objective lens; 3-OCT unit; 4-guidance light source; 5-light splitting unit; 6-optical zoom unit; 7-surgical microscope unit; 8-processing control unit; 9-display unit; 10-surgical lighting unit; 301-sweep-frequency laser; 302-first coupler; 303-wavelength division multiplexer; 304-first collimator; 305-two-dimensional galvanometer scanner; 306-second collimator; 307-reflector; 308-third collimator; 309-second coupler; 310-balance detector; 501-dichroic mirror; 701-left imaging lens; 702-right imaging lens; 703-left camera; and 704-right camera.

### DETAILED DESCRIPTION

The present disclosure will be further illustrated in detail below in combination with embodiments, so that those skilled in the art can implement accordingly with reference to the text of the specification.

It should be understood that terms such as "have", "comprise" and "include" used herein are not exclusive of existence or addition of one or more other elements or a combination thereof

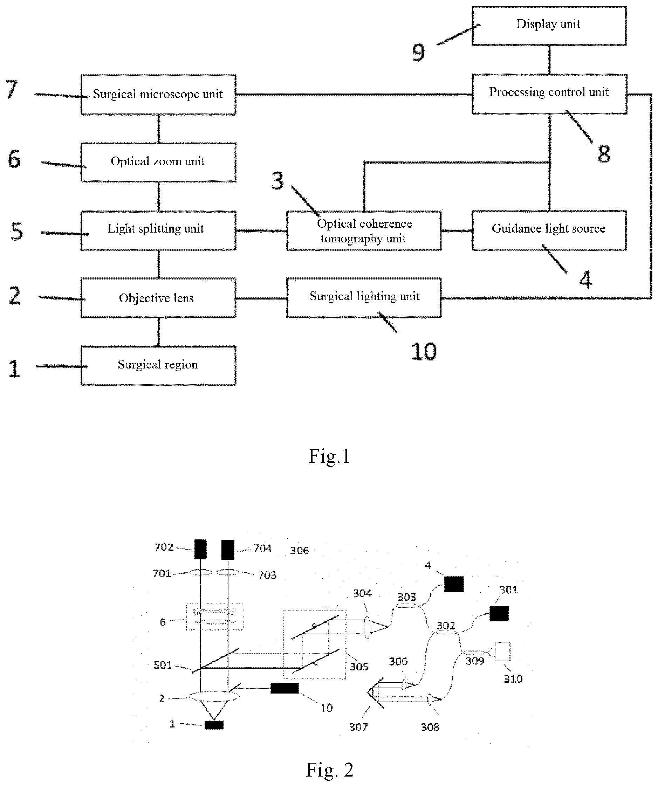

As shown in Figs. 1-2, an OCT augmented reality-based surgical microscope imaging system in an embodiment includes:
a surgical microscope unit 7, configured to acquire a two-dimensional microscopic image of a surgical region 1;
an OCT unit 3, configured to acquire an OCT three-dimensional image of the surgical region 1;
a guidance light source 4, which can be captured by cameras of the surgical microscope unit 7 and is configured to project, into the surgical region, a guidance light spot synchronized with an OCT scanning light source of the OCT unit 3, light emitted by the guidance light source 4 being coaxial with OCT light;
a processing control unit 8, configured to acquire the two-dimensional microscopic image and the OCT three-dimensional image of the surgical region 1, and an image obtained by fusing the two-dimensional microscopic image and the OCT three-dimensional image of the surgical region 1; and
a display unit 9, configured to output and display a result of the processing control unit 8 to carry out navigation for surgery.

The system further includes a surgical lighting unit 10, and an objective lens 2, a light splitting unit 5 and an optical zoom unit 6 sequentially arranged along an imaging optical path of the surgical microscope unit 7;
the surgical lighting unit 10 is configured to provide lighting light for the surgical region 1, and the lighting light reflected by the surgical region 1 enters the surgical microscope unit 7 after sequentially passing through the objective lens 2, the light splitting unit 5 and the optical zoom unit 6 so as to implement two-dimensional microscopic imaging of the surgical region 1; and
light emitted by the guidance light source 4 and the OCT scanning light source of the OCT unit 3 reaches the surgical region 1 after sequentially passing through the light splitting unit 5 and the objective lens 2, and OCT scanning light reflected by the surgical region 1 backtracks to the OCT unit 3 to implement OCT three-dimensional imaging; and after guidance light reflected by the surgical region 1 passes through the light splitting unit 5, one portion of the guidance light enters the OCT unit 3, while the other portion of the guidance light enters the surgical microscope unit 7.

The surgical microscope unit 7 is configured to carry out two-dimensional imaging on the surgical region 1 via the objective lens 2, and the OCT unit 3 is configured to carry out two-dimensional scanning on the surgical region 1 via the objective lens 2 and implement three-dimensional tomography imaging of the surgical region 1 by the longitudinal tomography capacity of OCT. The surgical microscope unit 7 and the OCT unit 3 are configured to carry out coaxial imaging via an on-axis region of the objective lens 2. The guidance light source 4 is configured to project, into the surgical region 1, a guidance spot light source coaxial with the OCT scanning, and finally, can be captured by the cameras of the surgical microscope unit 7. The light splitting unit 5 is configured to implement light splitting and matching of light output by the microscope unit, the OCT unit 3 and the guidance light source 4 so as to implement coupling and separation of light with different wavelengths. The optical zoom unit 6 is configured for optical amplification of the surgical microscope unit 7 so as to achieve different imaging resolutions. The processing control unit 8 coordinates work of components, and acquires navigation information. The navigation information includes the two-dimensional microscopic image (a high-resolution surface microscopic result of the surgical region 1) and the OCT three-dimensional image (including an OCT three-dimensional imaging result of surgical instruments and tissue internal structures) of the surgical region 1, and an imaging result obtained by fusing the two-dimensional microscopic image and the OCT three-dimensional image. An output unit is a stereoscopic polarization optical display, and can output both a left path of navigation information and a right path of navigation information so as to carry out three-dimensional real-time monitoring on the surgical instruments and the tissues of the surgical region 1 in the surgery process. The surgical lighting unit carries out uniform lighting on the surgical region 1 via the objective lens 2.

The surgical microscope unit 7 is a binocular surgical microscope unit 7 and includes imaging lenses and cameras, the imaging lenses include a left imaging lens 701 and a right imaging lens 702, and the cameras include a left camera 703 and a right camera 704, wherein the left imaging lens 701 and the left camera 703 correspondingly constitute a left microscopic imaging module, and the right imaging lens 702 and the right camera 704 correspondingly constitute a right microscopic imaging module. The surgical microscope unit 7 is used for carrying out two-dimensional imaging on the surgical region 1 via the objective lens 2, and two-dimensional imaging is carried out on the surgical region 1 by the two cameras.

In the embodiment, the surgical microscope unit 7 is configured to carry out large-viewing-field two-dimensional imaging on a region where surgery is carried out, and the surgical region 1 can be, through the cameras, converted into a digital image which is displayed by the display unit 9. For example, light emitted by the surgical lighting unit 10 uniformly irradiates the surgical region 1 after passing through a rangefinder of the objective lens 2, and after being reflected by the surgical region 1, a lighting beam enters the surgical microscope unit 7 through a main axis of the objective lens 2, the light splitting unit 5 and the optical zoom unit 6, so that a surgery operator can directly observe, on the display unit 9, a binocular stereoscopic visual image obtained after image fusion of the surgical region 1.

In the embodiment, the OCT unit 3 is configured to carry out two-dimensional scanning on the surgical region 1 and obtain the three-dimensional image of the surgical region 1 by the longitudinal tomography capacity of the OCT technology. For example, an imaging beam of the OCT unit 3 transmits via the objective lens 2 to reach the surgical region 1, and after being reflected by the surgical region 1, the imaging beam passes through the objective lens 2 and the light splitting unit 5 and then returns to the OCT unit 3. The OCT unit 3 can convert a detected interference signal into an electrical signal, three-dimensional reconstruction is carried out in the processing control unit, and after registration is respectively carried out with double paths of viewing angles of a microscope, views of left and right eyes are acquired, so that the views are fused with the binocular image acquired by the surgical microscope unit 7. After processing, double-path output is carried out in the display unit 9, and the surgery operator can synchronously observe, in the display unit 9, the microscopic image with a stereoscopic perception effect and the OCT three-dimensional tomography image of the surgical region 1 so as to locate positions of the surgical instruments and the tissue internal structures in the three-dimensional space.

In a further preferred embodiment, the light splitting unit 5 is a dichroic mirror 501 which carries out total reflection on the light of the OCT unit 3, carries out semi-transmission and semi-reflection on the light of the guidance light source 4, and carries out total transmission on the light of the surgical lighting unit 10;
the OCT unit 3 includes the OCT scanning light source (which specifically is a sweep-frequency laser 301 in the embodiment), a first coupler 302, a wavelength division multiplexer 303, a first collimator 304, a two-dimensional galvanometer scanner 305, a second collimator 306, a reflector 307, a third collimator 308, a second coupler 309 and a balance detector 310;
an OCT scanning beam emitted by the OCT scanning light source is split into two paths of light via the first coupler 302, one path of light is sample light, and the other path of light is reference light;
guidance light emitted by the guidance light source 4 and the sample light are converged via the wavelength division multiplexer 303, then pass through the first collimator 304 together to become incident to the two-dimensional galvanometer scanner 305 to be deflected, and then are focused into the surgical region 1 by the objective lens 2 after being reflected by the dichroic mirror 501;
both the sample light and one portion of guidance light reflected by the surgical region 1 return along an original path after being reflected by the dichroic mirror 504, and reach one end of the second coupler 309 after passing through the first coupler 302; the other portion of guidance light reflected by the surgical region 1 transmits through the dichroic mirror 501 after passing through the objective lens 2, passes through the optical zoom unit 6, and then respectively passes through the left imaging lens 701 and the right imaging lens 702 to respectively enter the left camera 703 and the right camera 704;
the reference light emergent after passing through the first coupler 302 sequentially passes through the second collimator 306, the reflector 307 and the third collimator 308 to reach said one end of the second coupler 309, and enters the second coupler 309 together with the sample light and said one portion of guidance light that have been reflected by the surgical region 1 and reached said one end of the second coupler 309, and the reference light undergoes interference with the sample light and said one portion of guidance light before being received by the balance detector 310, and finally, a detection result is output to the processing control unit 8 so as to implement OCT three-dimensional imaging;
after a lighting beam emitted by the surgical lighting unit 10 irradiates the surgical region 1, the lighting light and the other portion of guidance light reflected by the surgical region 1 transmit through the dichroic mirror 501, then pass through the optical zoom unit 6 and subsequently enter the left microscopic imaging module and the right microscopic imaging module, and finally, an imaging signal is output to the processing control unit 8 so as to implement two-dimensional microscopic imaging of the surgical region 1; and
the processing control unit 8 carries out registration and fusion on the two-dimensional microscopic image and the OCT three-dimensional image of the surgical region 1, and a fused image is displayed and output by the display unit 9 so as to carry out navigation for surgery.

The display unit 9 is a polarized light display screen with a stereoscopic visual effect, and is configured to respectively output respective fused images of both the left visual pathway and right visual pathway (an image obtained by fusing the two-dimensional microscopic image from the left microscopic imaging module and the OCT three-dimensional image, and an image obtained by fusing the two-dimensional microscopic image from the right microscopic imaging module and the OCT three-dimensional image).

The guidance light source 4 and the lighting light unit may be controlled by the processing control unit, and the light intensity is controlled, so that the cameras can acquire the image, having the optimal effect, of the surgical region 1 as required, or can simultaneously distinguish the image of the surgical region 1 from the image of the guidance light spot.

The present disclosure further discloses an OCT augmented reality-based surgical microscope imaging method. The method uses the OCT augmented reality-based surgical microscope imaging system of the above-mentioned embodiments to carry out imaging.

A specific operation method for carrying out acquisition and fusion on a microscope image and an OCT three-dimensional image is as follows.

OCT signal processing includes: an interference signal acquired from a balance detector 310 is subjected to demodulation (including mean subtraction, windowing, inverse fast Fourier transform and mod value acquisition), and then intensity information of the interference signal in a depth domain is obtained. Then internal structure information of tissues of the surgical region 1 can be extracted according to surgery demands, wherein structural image mapping includes: logarithmic mapping, brightness, contrast mapping and 8-bit grey-scale map mapping. Based on the structural image, invalid information which influences the internal structure display, such as a nontransparent surface layer, is filtered out, valid surgical information, such as surgical instruments under tissues and target tissues, in an OCT image is reserved, and a new acquired image is used for input of subsequent three-dimensional reconstruction.

After being subjected to said processing, OCT original data is respectively fused with the images acquired by the left camera 703 and the right camera 704, wherein a to-be-fused OCT image needs to be registered in advance, and needs to be registered again each time when parameters related to surgical microscope navigation system imaging, such as the OCT scanning direction and the microscope imaging magnification, are changed.

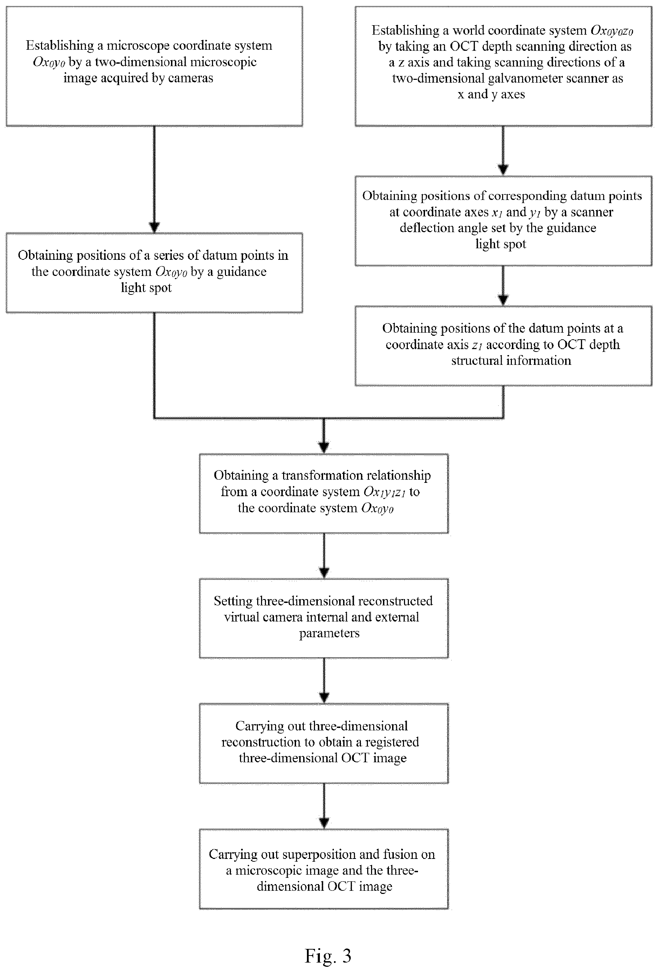

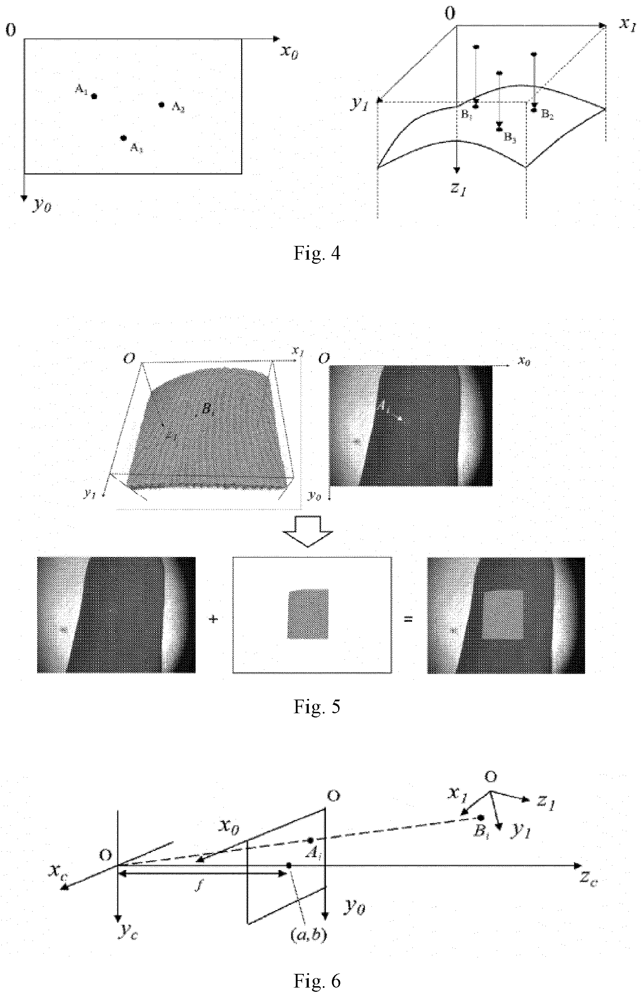

With reference to Fig. 3, the OCT augmented reality-based surgical microscope imaging method according to the embodiment includes the following steps:
S1: adjusting the output intensity and focus positions of a surgical lighting unit 10 and a guidance light source 4 to enable cameras of a surgical microscope unit 7 to clearly observe a surgical region 1 and a guidance light spot, and acquiring a microscopic image of the surgical region 1;
S2: establishing a microscope two-dimensional Cartesian coordinate system *Ox₀y₀* by taking a two-dimensional plane of the microscopic image acquired by the cameras as x and y axes, obtaining coordinates of the guidance light spot in the microscope coordinate system according to a position of the guidance light spot in the image, and using the obtained coordinates as a datum point; and changing, in an OCT three-dimensional scanning region, a deflection angle of a two-dimensional galvanometer scanner, acquiring coordinates of a series of different datum points to be marked as {A1, A2 ... An}, as shown in the left portion of Fig. 4 (only showing A1, A2 and A3);
S3: establishing a three-dimensional Cartesian coordinate system *Ox₀y₀z₀,* named an OCT coordinate system, by taking a plurality of pieces of continuous OCT slicing data at adjacent positions as volume data, taking an OCT depth scanning direction as a z axis and taking scanning directions of the two-dimensional galvanometer scanner as *x* and *y* axes; carrying out primary OCT three-dimensional scanning on an imaging region, wherein due to the fact that a scanner deflection angle corresponding to a projection position of guidance light in the step S2 is known, coordinate values of *x₁* and *y₁,* corresponding to the position of the guidance light spot of the step S2, in the OCT coordinate system is also known, finding a boundary where the guidance light spot is located according to an OCT structure, thus acquiring coordinate values of z₁ of the guidance light spot of the step S2 in the OCT coordinate system, and finally, obtaining coordinates {*B₁, B₂* ... *Bₙ*} in the OCT coordinate system corresponding to the datum points {*A₁,* A₂ ... *Aₙ*} in the microscope two-dimensional Cartesian coordinate system *Ox₀y₀,* as shown in the right portion of Fig. 4 (only showing B1, B2 and B3);
S4: carrying out fitting on {*A₁,* A₂ ... *Aₙ*} and {*B₁, B₂* ... *Bₙ*} to obtain a transformation relationship from the OCT coordinate system to the microscope two-dimensional Cartesian coordinate system, which is a homography matrix corresponding to coordinate transformation, calibrating the cameras to obtain internal parameters of the cameras, and carrying out matrix operation to obtain external parameters of the cameras;
S5: adjusting the intensity of the surgical lighting unit 10 to the conventional surgical microscope imaging brightness, and simultaneously starting to carry out OCT three-dimensional scanning on the surgical region 1;
S6: setting virtual camera parameters of an OCT three-dimensional reconstructed portion according to the microscope external parameters obtained in the step S4 so as to obtain a registered OCT three-dimensional reconstructed image, and finally, carrying out superposition on the registered OCT three-dimensional reconstructed image and the microscopic image of the surgical region 1 to complete virtual-and-real-image fusion display; and
S7: repeating the step S6 as OCT scanning continuously updates the input volume data, reconstructing all two-dimensional structural images to form a three-dimensional tomography model of the surgical region 1, and carrying out display by a display unit 9 so as to implement real-time augmentation on the microscopic image of the surgical region 1.

When the above-mentioned steps are performed, corresponding to the left camera 703 and the right camera 704, respective microscope coordinate systems need to be respectively established, and then registration and fusion are carried out with an OCT image respectively so as to obtain an image fusion result with a binocular stereoscopic visual effect.

When the position of the datum point is set, the defection angle of the two-dimensional galvanometer scanner 305 is a value during OCT three-dimensional scanning, instead of a random value in a scannable range.

The above-mentioned steps need to be carried out again when the parameters related to system imaging (such as the OCT scanning direction and the microscope imaging magnification) are changed.

The OCT two-dimensional image participating in three-dimensional reconstruction only includes valid information, such as surgical instruments under tissues and target tissues, and cannot be shielded by invalid information above, such as a nontransparent tissue surface layer; and the image is extracted from the OCT two-dimensional structural image.

A number of the datum points required in the step S2 is n, n≥6.

Fig. 5 shows a chart flow of fusion of a finger microscopic image and an OCT image acquired by single cameras, and in the process of acquiring the microscopic image, guidance light is turned on, and the galvanometer scanner is in a static state. The upper portion of Fig. 5 shows a three-dimensional OCT image in the OCT coordinate system from left to right and an image acquired by the camera in the microscope coordinate system, where Ai represents microscope coordinates of the guidance light spot, and Bi represents OCT coordinates of the guidance light spot. The lower portion of Fig. 5 shows a superposition process of the microscopic image and the registered three-dimensional OCT image, and shows a result after fusion.

The above-mentioned step S4 is the virtual-and-real registration process in augmented reality, and in an embodiment, the adopted specific principle and method are as follows:
as shown in Fig. 4, *Ox₁y₁z₁* is an OCT coordinate system and is used as a world coordinate system, i.e., an absolute coordinate system of the objective world; a three-dimensional Cartesian coordinate system *Ox_{c}y_{c}z_{c}* is a camera coordinate system, an origin is located at an optical center of a video camera, and *Z_{c}* coincides with an optical axis; and *Ox₀y₀* is a microscope coordinate system. With reference to Fig. 6, imaging transformation from *Ox₁y₁z₁* to *Ox₀y₀* can be described as follows:
a transformation relationship from the OCT coordinate system to the camera coordinate system is X_{c}:
   ${X}_{c} = \left[\begin{matrix}{x}_{c} \\ {y}_{c} \\ {z}_{c} \\ 1\end{matrix}\right] = \left[\begin{matrix}R & t \\ 0 & 1\end{matrix}\right] \left[\begin{matrix}{x}_{1} \\ {y}_{1} \\ {z}_{1} \\ 1\end{matrix}\right] = {T}_{w} \left[\begin{matrix}{x}_{1} \\ {y}_{1} \\ {z}_{1} \\ 1\end{matrix}\right]$ (1)
where R represents a rotation matrix in which rotation transformation is recorded, t represents a three-dimensional translation vector, and T_{w} includes a position and a direction of the camera relative to the world coordinate system, and thus are called as external parameters of the camera.

A transformation relationship from the camera coordinate system to the microscope coordinate system is Z₀:
${Z}_{0} = \left[\begin{matrix}{x}_{0} \\ {y}_{0} \\ 1\end{matrix}\right] = \left[\begin{matrix}\frac{1}{{d}_{x}} & 0 & a \\ 0 & \frac{1}{{d}_{y}} & b \\ 0 & 0 & 0\end{matrix}\right] \left[\begin{matrix}f & 0 & 0 & 0 \\ 0 & f & 0 & 0 \\ 0 & 0 & 1 & 0\end{matrix}\right] \left[\begin{matrix}{x}_{c} \\ {y}_{c} \\ {z}_{c} \\ 1\end{matrix}\right] = \left[\begin{matrix}{α}_{x} & 0 & a & 0 \\ 0 & {α}_{y} & b & 0 \\ 0 & 0 & 1 & 0\end{matrix}\right] \left[\begin{matrix}{x}_{c} \\ {y}_{c} \\ {z}_{c} \\ 1\end{matrix}\right] = K \left[\begin{matrix}{x}_{c} \\ {y}_{c} \\ {z}_{c} \\ 1\end{matrix}\right]$ (2)
where *dx* and *dy* represent physical distances of a pixel point of the microscope image on the *x* and *y* axes, *f* represents a distance from the microscope plane to the camera focal plane, *a* and *b* represent coordinates of a principal point of the camera in the microscope coordinate system, *αₓ* and *α_{y}* represent a height-to-width ratio of a pixel, *αₓ*=f/*dx,* and *α_{y}*=f/*dy.* K is only related to an internal structure of the camera, and thus is an internal parameter of the camera. A transformation relationship from the OCT coordinate system to the microscope coordinate system can be obtained from the formula (1) and the formula (2) as follows:
$\left[\begin{matrix}{x}_{0} \\ {y}_{0} \\ 1\end{matrix}\right] = {KT}_{w} \left[\begin{matrix}{x}_{1} \\ {y}_{1} \\ {z}_{1} \\ 1\end{matrix}\right] = P \left[\begin{matrix}{x}_{1} \\ {y}_{1} \\ {z}_{1} \\ 1\end{matrix}\right]$ (3)

For each pair of points *Aᵢ* and *Bᵢ* in the step S4, the following formula is met:
${A}_{i} = {PB}_{i} , and P = {KT}_{w} = K \left[\left.R\right|t\right]$ (4)
where, P represents a 3*4 matrix, and P can be solved through at least six pairs of *Aᵢ* and *Bᵢ*. P can be written as: $P = {KT}_{w} = K \left[\left.R\right|t\right] = \left[\left.KR\right|Kt\right] = \left[\left.M\right|Kt\right]$ (5)

The rotation matrix R is an orthogonal matrix, and thus the following formula is met: ${MM}^{T} = {KRR}^{T} {K}^{T} = {KK}^{T}$ (6)
where the superscript T represents matrix transposition; and in addition, K represents an upper triangular matrix, and thus, K and R can be solved. Moreover, t can be obtained by the following formula: $t = {K}^{- 1} {\left({P}_{14}{P}_{24}{P}_{34}\right)}^{T}$ (7)
where the subscripts of P represent the matrix row and column. So far, the external parameters T_{w} and the internal parameter K of the camera all have been solved, i.e., the transformation relationship from the OCT coordinate system to the microscope two-dimensional Cartesian coordinate system is obtained.

The above three-dimensional reconstruction operation flow includes: inputting a plurality of continuous OCT slicing data at the adjacent positions as the volume data into the three-dimensional reconstructed portion, and based on a volume rendering algorithm, reconstructing all two-dimensional structural images to form the three-dimensional tomography model of the surgical region 1. The double-path image fusion result is finally output by a stereoscopic polarization optical display.

Although the implementation solution of the present disclosure has been disclosed as above, it is not limited to application listed in the specification and the implementation modes and it totally can be applicable to various fields suitable for the present disclosure. For those skilled in the art, additional modifications can be easily implemented, and thus, the present disclosure is not limited to the specific details without departure from the claims.

## Claims

1. An optical coherence tomography (OCT) augmented reality-based surgical microscope imaging system, comprising:
a surgical microscope unit (7), configured to acquire a two-dimensional microscopic image of a surgical region (1);
an OCT unit (3), configured to acquire an OCT three-dimensional image of the surgical region (1);
a processing control unit (8), configured to acquire the two-dimensional microscopic image and the OCT three-dimensional image of the surgical region (1), and an image obtained by fusing the two-dimensional microscopic image and the OCT three-dimensional image of the surgical region (1);
a display unit (9), configured to output and display a result of the processing control unit (8) to carry out navigation for surgery; and
a guidance light source (4), which can be captured by the surgical microscope unit (7) and is configured to project, into the surgical region (1), a guidance light spot synchronized with an OCT scanning light source (301) of the OCT unit (3);
the OCT unit (3) comprises the OCT scanning light source (301), a first coupler (302), a wavelength division multiplexer (303), a first collimator (304), a two-dimensional galvanometer scanner (305), a second collimator (306), a reflector (307), a third collimator (308), a second coupler (309) and a balance detector (310);
an OCT scanning beam emitted by the OCT scanning light source (301) is split into two paths of light via the first coupler (302), one path of light is sample light, and the other path of light is reference light;
guidance light emitted by the guidance light source (4) and the sample light are converged via the wavelength division multiplexer (303), then pass through the first collimator (304) together to become incident to the two-dimensional galvanometer scanner (305) to be deflected, and then are focused into the surgical region (1) by an objective lens (2) after being reflected by a dichroic mirror (501);
both the sample light and one portion of guidance light reflected by the surgical region (1) return along an original path after being reflected by the dichroic mirror (501), and reach one end of the second coupler (309) after passing through the first coupler (302); the other portion of guidance light reflected by the surgical region (1) transmits through the dichroic mirror (501) after passing through the objective lens (2), passes through an optical zoom unit (6), and then respectively passes through a left imaging lens (701) and a right imaging lens (702) to respectively enter a left camera (703) and a right camera (704);
the reference light emergent after passing through the first coupler (302) sequentially passes through the second collimator (306), the reflector (307), and the third collimator (308) to reach said one end of the second coupler (309), and enters the second coupler (309) together with the sample light and said one portion of guidance light that have been reflected by the surgical region (1) and reached said one end of the second coupler (309), and the reference light undergoes interference with the sample light and said one portion of guidance light before being received by the balance detector (310), and finally, a detection result is output to the processing control unit (8) so as to implement OCT three-dimensional imaging;
after a lighting beam emitted by a surgical lighting unit (10) irradiates the surgical region (1), the lighting light and the other portion of guidance light reflected by the surgical region (1) transmit through the dichroic mirror (501), then pass through the optical zoom unit (6) and subsequently enter a left microscopic imaging module and a right microscopic imaging module, and finally, an imaging signal is output to the processing control unit (8) so as to implement two-dimensional microscopic imaging of the surgical region (1); and
the processing control unit (8) is configured to carry out registration and fusion on the two-dimensional microscopic image and the OCT three-dimensional image of the surgical region (1), and a fused image is displayed and output by the display unit (9) so as to carry out navigation for surgery.

2. The OCT augmented reality-based surgical microscope imaging system according to claim 1, further comprising a surgical lighting unit (10), and an objective lens (2), a light splitting unit (5) and an optical zoom unit (6) sequentially arranged along an imaging optical path of the surgical microscope unit (7), wherein
the surgical lighting unit (10) is configured to provide lighting light for the surgical region (1), and the lighting light reflected by the surgical region (1) enters the surgical microscope unit (7) after sequentially passing through the objective lens (2), the light splitting unit (5) and the optical zoom unit (6) so as to implement two-dimensional microscopic imaging of the surgical region (1);
light emitted by the guidance light source (4) and the OCT scanning light source (301) of the OCT unit (3) reaches the surgical region (1) after sequentially passing through the light splitting unit (5) and the objective lens (2), and OCT scanning light reflected by the surgical region (1) backtracks to the OCT unit (3) to implement OCT three-dimensional imaging; and after guidance light reflected by the surgical region (1) passes through the light splitting unit (5), one portion of the guidance light enters the OCT unit (3), while the other portion of the guidance light enters the surgical microscope unit (7).

3. The OCT augmented reality-based surgical microscope imaging system according to claim 2, wherein the surgical microscope unit (7) comprises imaging lenses and cameras, the imaging lenses include a left imaging lens (701) and a right imaging lens (702), and the cameras include a left camera (703) and a right camera (704), wherein the left imaging lens (701) and the left camera (703) correspondingly constitute a left microscopic imaging module, and the right imaging lens (702) and the right camera (704) correspondingly constitute a right microscopic imaging module.

4. The OCT augmented reality-based surgical microscope imaging system according to claim 3, wherein the light splitting unit (5) is a dichroic mirror (501) which carries out total reflection on the light of the OCT unit (3), carries out semi-transmission and semi-reflection on the light of the guidance light source (4), and carries out total transmission on the light of the surgical lighting unit (10).

5. The OCT augmented reality-based surgical microscope imaging system according to claim 1, wherein the display unit (9) is a polarized light display screen with a stereoscopic visual effect, and is configured to respectively output an image obtained by fusing the two-dimensional microscopic image from the left microscopic imaging module and the OCT three-dimensional image and output an image obtained by fusing the two-dimensional microscopic image from the right microscopic imaging module and the OCT three-dimensional image.

6. An OCT augmented reality-based surgical microscope imaging method, using the system according to any one of claims 1-5 to carry out imaging, and comprising the following steps:
S1: adjusting the output intensity and focus positions of a surgical lighting unit (10) and a guidance light source (4) to enable cameras of a surgical microscope unit (7) to clearly observe a surgical region (1) and a guidance light spot, and acquiring a microscopic image of the surgical region (1);
S2: establishing a microscope two-dimensional Cartesian coordinate system *Ox₀y₀* by taking a two-dimensional plane of the microscopic image acquired by the cameras as x and y axes and taking the upper left corner of the microscopic image as an origin, obtaining coordinates of the guidance light spot in the microscope coordinate system according to a position of the guidance light spot in the image, and using the obtained coordinates as a datum point; and changing, in an OCT three-dimensional scanning region, a deflection angle of a two-dimensional galvanometer scanner (305), acquiring coordinates of a series of different datum points to be marked as {*A₁,* A₂ ... Aₙ};
S3: establishing a three-dimensional Cartesian coordinate system *Ox₀y₀z₀,* named an OCT coordinate system, by taking a plurality of pieces of continuous OCT slicing data at adjacent positions as volume data, taking an OCT depth scanning direction as a z axis and taking scanning directions of the two-dimensional galvanometer scanner (305) as x and y axes; carrying out primary OCT three-dimensional scanning on an imaging region, wherein, due to the fact that a scanner deflection angle corresponding to a projection position of guidance light in the step S2 is known, coordinate values of *x₁* and *y₁,* corresponding to the position of the guidance light spot of the step S2, in the OCT coordinate system is also known, finding a boundary where the guidance light spot is located according to an OCT structure, thus acquiring coordinate values of z₁ of the guidance light spot of the step S2 in the OCT coordinate system, and finally, obtaining coordinates {*B₁, B₂* ... *Bₙ*} in the OCT coordinate system corresponding to the datum points {*A₁,* A₂ ... *Aₙ*} in the microscope two-dimensional Cartesian coordinate system *Ox₀y₀*;
S4: carrying out fitting on {*A₁,* A₂ ... *Aₙ*} and {*B₂, B₂* ... *Bₙ*} to obtain a transformation relationship $\left[\begin{matrix}{x}_{0} \\ {y}_{0} \\ 1\end{matrix}\right] = {KT}_{w} \left[\begin{matrix}{x}_{1} \\ {y}_{1} \\ {z}_{1} \\ 1\end{matrix}\right] = P \left[\begin{matrix}{x}_{1} \\ {y}_{1} \\ {z}_{1} \\ 1\end{matrix}\right]$ from the OCT coordinate system to the microscope twodimensional Cartesian coordinate system, which is a homography matrix corresponding to coordinate transformation, calibrating the cameras to obtain internal parameters K of the cameras, and carrying out matrix operation to obtain external parameters T_{w} of the cameras;
S5: adjusting the intensity of the surgical lighting unit (10), and simultaneously starting to carry out OCT three-dimensional scanning on the surgical region (1);
S6: obtaining a registered OCT three-dimensional reconstructed image according to the parameters of the cameras obtained in the step S4, and finally, carrying out superposition on the registered OCT three-dimensional reconstructed image and the microscopic image of the surgical region (1) to complete virtual-and-real-image fusion display; and
S7: repeating the step S6 as OCT scanning continuously updates the input volume data, reconstructing all two-dimensional structural images to form a three-dimensional tomography model of the surgical region (1), and carrying out display by a display unit (9) so as to implement real-time augmentation on the microscopic image of the surgical region (1).

7. The OCT augmented reality-based surgical microscope imaging method according to claim 6, comprising: establishing respective microscope coordinate systems corresponding to the left camera (703) and the right camera (704) respectively, and then respectively carrying out registration and fusion with an OCT image.

8. The OCT augmented reality-based surgical microscope imaging method according to claim 6, wherein when the position of the datum point is set, the defection angle of the two-dimensional galvanometer scanner (305) is a value during OCT three-dimensional scanning, instead of a random value in a scannable range.

9. The OCT augmented reality-based surgical microscope imaging method according to claim 6, wherein a number of the datum points required in the step S2 is n, and n≥6.

## Patentansprüche

1. Bildgebungssystem für ein chirurgisches Mikroskop auf Basis von erweiterter Realität mit optischer Kohärenztomografie (OCT), umfassend:
eine chirurgische Mikroskopeinheit (7), die dazu eingerichtet ist, ein zweidimensionales mikroskopisches Bild eines Operationsbereichs (1) zu erfassen;
eine OCT-Einheit (3), die dazu eingerichtet ist, ein dreidimensionales OCT-Bild des Operationsbereichs (1) zu erfassen;
eine Verarbeitungssteuereinheit (8), die dazu eingerichtet ist, das zweidimensionale mikroskopische Bild und das dreidimensionale OCT-Bild des Operationsbereichs (1) sowie ein durch Fusion des zweidimensionalen mikroskopischen Bildes und des dreidimensionalen OCT-Bildes des Operationsbereichs (1) erhaltenes Bild zu erfassen;
eine Anzeigeeinheit (9), die dazu eingerichtet ist, ein Ergebnis der Verarbeitungssteuereinheit (8) auszugeben und anzuzeigen, um eine Navigation bei einem chirurgischen Eingriff durchzuführen; und
eine Führungslichtquelle (4), die in der Lage ist, von der chirurgischen Mikroskopeinheit (7) erfasst zu werden, und dazu eingerichtet ist, einen mit einer OCT-Abtastlichtquelle (301) der OCT-Einheit (3) synchronisierten Führungslichtfleck in den Operationsbereich (1) zu projizieren;
wobei die OCT-Einheit (3) die OCT-Abtastlichtquelle (301), einen ersten Koppler (302), einen Wellenlängenmultiplexer (303), einen ersten Kollimator (304), einen zweidimensionalen Galvanometerscanner (305), einen zweiten Kollimator (306), einen Reflektor (307), einen dritten Kollimator (308), einen zweiten Koppler (309) und einen balancierten Detektor (310) umfasst;
wobei ein von der OCT-Abtastlichtquelle (301) emittierter OCT-Abtaststrahl mittels des ersten Kopplers (302) in zwei Lichtpfade aufgeteilt wird, wobei der eine Lichtpfad Probenlicht und der andere Lichtpfad Referenzlicht ist;
wobei von der Führungslichtquelle (4) emittiertes Führungslicht und das Probenlicht mittels des Wellenlängenmultiplexers (303) zusammengeführt werden, anschließend gemeinsam den ersten Kollimator (304) durchlaufen, auf den zweidimensionalen Galvanometerscanner (305) einfallen und von diesem abgelenkt werden und anschließend nach einer Reflexion an einem dichroitischen Spiegel (501) durch eine Objektivlinse (2) in den Operationsbereich (1) fokussiert werden;
wobei sowohl das von dem Operationsbereich (1) reflektierte Probenlicht als auch ein Teil des von dem Operationsbereich (1) reflektierten Führungslichts nach einer Reflexion an dem dichroitischen Spiegel (501) entlang des ursprünglichen Lichtwegs zurücklaufen und nach Durchlaufen des ersten Kopplers (302) ein Ende des zweiten Kopplers (309) erreichen, während der andere Teil des von dem Operationsbereich (1) reflektierten Führungslichts nach Durchlaufen der Objektivlinse (2) den dichroitischen Spiegel (501) durchtritt, eine optische Zoomeinheit (6) durchläuft und anschließend durch eine linke Abbildungslinse (701) und eine rechte Abbildungslinse (702) hindurchtritt, um in eine linke Kamera (703) und eine rechte Kamera (704) einzutreten;
wobei das nach Durchlaufen des ersten Kopplers (302) austretende Referenzlicht nacheinander den zweiten Kollimator (306), den Reflektor (307) und den dritten Kollimator (308) durchläuft, um das genannte Ende des zweiten Kopplers (309) zu erreichen, und zusammen mit dem Probenlicht und dem genannten Teil des Führungslichts, die von dem Operationsbereich (1) reflektiert worden sind das genannte Ende des zweiten Kopplers (309) erreicht haben, in den zweiten Koppler (309) eintritt, und wobei das Referenzlicht mit dem Probenlicht und dem genannten Teil des Führungslichts interferiert, bevor es von dem balancierten Detektor (310) empfangen wird, und schließlich ein Detektionsergebnis an die Verarbeitungssteuereinheit (8) ausgegeben wird, um eine dreidimensionale OCT-Bildgebung zu realisieren;
wobei, nachdem ein von einer Operationsbeleuchtungseinheit (10) emittierter Beleuchtungsstrahl den Operationsbereich (1) bestrahlt hat, das von dem Operationsbereich (1) reflektierte Beleuchtungslicht und der andere Teil des von dem Operationsbereich (1) reflektierten Führungslichts den dichroitischen Spiegel (501) durchtreten, anschließend die optische Zoomeinheit (6) durchlaufen und danach in ein linkes mikroskopisches Bildgebungsmodul und ein rechtes mikroskopisches Bildgebungsmodul eintreten, und wobei schließlich ein Bildsignal an die Verarbeitungssteuereinheit (8) ausgegeben wird, um eine zweidimensionale mikroskopische Bildgebung des Operationsbereichs (1) zu realisieren; und
wobei die Verarbeitungssteuereinheit (8) dazu eingerichtet ist, eine Registrierung und Fusion des zweidimensionalen mikroskopischen Bildes und des dreidimensionalen OCT-Bildes des Operationsbereichs (1) durchzuführen, wobei ein fusioniertes Bild von der Anzeigeeinheit (9) angezeigt und ausgegeben wird, um eine Navigation bei einem chirurgischen Eingriff durchzuführen.

2. Bildgebungssystem für ein chirurgisches Mikroskop auf Basis von erweiterter Realität mit optischer Kohärenztomografie (OCT) nach Anspruch 1, ferner umfassend eine Operationsbeleuchtungseinheit (10) sowie eine Objektivlinse (2), eine Strahlteilereinheit (5) und eine optische Zoomeinheit (6), die in dieser Reihenfolge entlang eines Abbildungsstrahlengangs der chirurgischen Mikroskopeinheit (7) angeordnet sind,
wobei die Operationsbeleuchtungseinheit (10) dazu eingerichtet ist, Beleuchtungslicht für den Operationsbereich (1) bereitzustellen, und das von dem Operationsbereich (1) reflektierte Beleuchtungslicht nach dem aufeinanderfolgenden Durchlaufen der Objektivlinse (2), der Strahlteilereinheit (5) und der optischen Zoomeinheit (6) in die chirurgische Mikroskopeinheit (7) eintritt, um eine zweidimensionale mikroskopische Bildgebung des Operationsbereichs (1) zu realisieren;
wobei das von der Führungslichtquelle (4) und der OCT-Abtastlichtquelle (301) der OCT-Einheit (3) emittierte Licht nach dem aufeinanderfolgenden Durchlaufen der Strahlteilereinheit (5) und der Objektivlinse (2) den Operationsbereich (1) erreicht und von dem Operationsbereich (1) reflektiertes OCT-Abtastlicht entlang des ursprünglichen Lichtwegs zu der OCT-Einheit (3) zurückläuft, um eine dreidimensionale OCT-Bildgebung zu realisieren; und wobei, nachdem das von dem Operationsbereich (1) reflektierte Führungslicht die Strahlteilereinheit (5) durchlaufen hat, ein Teil des Führungslichts in die OCT-Einheit (3) eintritt, während der andere Teil des Führungslichts in die chirurgische Mikroskopeinheit (7) eintritt.

3. Bildgebungssystem für ein chirurgisches Mikroskop auf Basis von erweiterter Realität mit optischer Kohärenztomografie (OCT) nach Anspruch 2, wobei die chirurgische Mikroskopeinheit (7) Abbildungslinsen und Kameras umfasst, und wobei die Abbildungslinsen eine linke Abbildungslinse (701) und eine rechte Abbildungslinse (702) umfassen und die Kameras eine linke Kamera (703) und eine rechte Kamera (704) umfassen, und wobei die linke Abbildungslinse (701) und die linke Kamera (703) gemeinsam ein linkes mikroskopisches Bildgebungsmodul bilden und die rechte Abbildungslinse (702) und die rechte Kamera (704) gemeinsam ein rechtes mikroskopisches Bildgebungsmodul bilden.

4. Bildgebungssystem für ein chirurgisches Mikroskop auf Basis von erweiterter Realität mit optischer Kohärenztomografie (OCT) nach Anspruch 3, wobei die Strahlteilereinheit (5) ein dichroitischer Spiegel (501) ist, der das Licht der OCT-Einheit (3) vollständig reflektiert, das Licht der Führungslichtquelle (4) zur Hälfte transmittiert und zur Hälfte reflektiert und das Licht der Operationsbeleuchtungseinheit (10) vollständig transmittiert.

5. Bildgebungssystem für ein chirurgisches Mikroskop auf Basis von erweiterter Realität mit optischer Kohärenztomografie (OCT) nach Anspruch 1, wobei die Anzeigeeinheit (9) ein Polarisationsanzeigeschirm mit einem stereoskopischen visuellen Effekt ist und dazu eingerichtet ist, ein durch Fusion des zweidimensionalen mikroskopischen Bildes aus dem linken mikroskopischen Bildgebungsmodul mit dem dreidimensionalen OCT-Bild erhaltenes Bild sowie ein durch Fusion des zweidimensionalen mikroskopischen Bildes aus dem rechten mikroskopischen Bildgebungsmodul mit dem dreidimensionalen OCT-Bild erhaltenes Bild auszugeben.

6. Bildgebungsverfahren für ein chirurgisches Mikroskop auf Basis von erweiterter Realität mit optischer Kohärenztomografie (OCT), wobei zur Bildgebung das System nach einem der Ansprüche 1 bis 5 verwendet wird und das Verfahren die folgenden Schritte umfasst:
S1: Einstellen der Ausgangsintensitäten und der Fokuspositionen einer Operationsbeleuchtungseinheit (10) und einer Führungslichtquelle (4), sodass Kameras einer chirurgischen Mikroskopeinheit (7) in der Lage ist, einen Operationsbereich (1) und einen Führungslichtfleck deutlich zu erfassen, und Erfassen eines mikroskopischen Bildes des Operationsbereichs (1);
S2: Erstellen eines zweidimensionalen kartesischen Mikroskopkoordinatensystems Ox₀y₀, indem die zweidimensionale Ebene des von den Kameras erfassten mikroskopischen Bildes als x- und y-Achsen und die linke obere Ecke des mikroskopischen Bildes als Ursprung festgelegt werden, Ermitteln von Koordinaten des Führungslichtflecks in dem Mikroskopkoordinatensystem anhand einer Position des Führungslichtflecks in dem Bild und Verwenden der ermittelten Koordinaten als Referenzpunkt; und Ändern eines Ablenkwinkels eines zweidimensionalen Galvanometerscanners (305) in einem dreidimensionalen OCT-Scanbereich und Erfassen der Koordinaten einer Reihe unterschiedlicher Referenzpunkte, die als {A₁, A₂, ..., Aₙ} bezeichnet werden;
S3: Erstellen eines dreidimensionalen kartesischen Koordinatensystems Ox₀y₀z₀, das als OCT-Koordinatensystem bezeichnet wird, indem mehrere aufeinanderfolgende OCT-Schnittdatensätze an benachbarten Positionen als Volumendaten, eine OCT-Tiefenscanrichtung als z-Achse und Scanrichtungen des zweidimensionalen Galvanometerscanners (305) als x- und y-Achse festgelegt werden; Durchführen eines ersten dreidimensionalen OCT-Scans eines Bildgebungsbereichs, wobei aufgrund dessen, dass ein Ablenkwinkel des Scanners, der einer Projektionsposition des Führungslichts in Schritt S2 entspricht, bekannt ist, auch die Koordinatenwerte x₁ und y₁, die der Position des Führungslichtflecks aus Schritt S2 in dem OCT-Koordinatensystem entsprechen, bekannt sind, Bestimmen einer Grenzfläche, an der sich der Führungslichtfleck befindet, anhand einer OCT-Struktur, wodurch der Koordinatenwert z₁ des Führungslichtflecks aus Schritt S2 in dem OCT-Koordinatensystem ermittelt wird, und schließlich Ermitteln der Koordinaten {B₁, B₂, ..., Bₙ} in dem OCT-Koordinatensystem, die den Referenzpunkten {A₁, A₂, ..., Aₙ} in dem zweidimensionalen kartesischen Mikroskopkoordinatensystem Ox₀y₀ entsprechen; S4: Durchführen einer Anpassung der Koordinaten {A₁, A₂, ..., Aₙ} und {B₁, B₂, ..., Bₙ}, um eine Transformationsbeziehung $\left[\begin{matrix}{x}_{0} \\ {y}_{0} \\ 1\end{matrix}\right] = {KT}_{w} \left[\begin{matrix}{x}_{1} \\ {y}_{1} \\ {z}_{1} \\ 1\end{matrix}\right] = P \left[\begin{matrix}{x}_{1} \\ {y}_{1} \\ {z}_{1} \\ 1\end{matrix}\right]$ von dem OCT-Koordinatensystem zu dem zweidimensionalen kartesischen Mikroskopkoordinatensystem zu erhalten, wobei die Transformationsbeziehung eine der Koordinatentransformation entsprechende Homografiematrix ist, Kalibrieren der Kameras, um interne Parameter K der Kameras zu erhalten, und Durchführen einer Matrixoperation, um externe Parameter T_{w} der Kameras zu erhalten;
S5: Einstellen der Intensität der Operationsbeleuchtungseinheit (10) und gleichzeitiges Beginnen eines dreidimensionalen OCT-Scans des Operationsbereichs (1);
S6: Erzeugen eines registrierten dreidimensionalen OCT-Rekonstruktionsbildes anhand der in Schritt S4 erhaltenen Parameter der Kameras und anschließendes Überlagern des registrierten dreidimensionalen OCT-Rekonstruktionsbildes mit dem mikroskopischen Bild des Operationsbereichs (1), um eine Fusionsanzeige eines virtuellen Bildes und eines realen Bildes zu vervollständigen; und
S7: Wiederholen von Schritt S6, während der OCT-Scan die eingegebenen Volumendaten fortlaufend aktualisiert, Rekonstruieren sämtlicher zweidimensionaler Strukturbilder zu einem dreidimensionalen Tomografiemodell des Operationsbereichs (1) und Anzeigen mittels einer Anzeigeeinheit (9), um eine Echtzeit-Erweiterung des mikroskopischen Bildes des Operationsbereichs (1) zu realisieren.

7. Bildgebungsverfahren für ein chirurgisches Mikroskop auf Basis von erweiterter Realität mit optischer Kohärenztomografie (OCT) nach Anspruch 6, umfassend: Erstellen eines der linken Kamera (703) entsprechenden Mikroskopkoordinatensystems und eines der rechten Kamera (704) entsprechenden Mikroskopkoordinatensystems sowie anschließendes jeweiliges Durchführen einer Registrierung und Fusion mit einem OCT-Bild.

8. Bildgebungsverfahren für ein chirurgisches Mikroskop auf Basis von erweiterter Realität mit optischer Kohärenztomografie (OCT) nach Anspruch 6, wobei beim Festlegen der Position des Referenzpunkts der Ablenkwinkel des zweidimensionalen Galvanometerscanners (305) ein während des dreidimensionalen OCT-Scans verwendeter Wert ist und kein beliebiger Wert innerhalb eines Scanbereichs ist.

9. Bildgebungsverfahren für ein chirurgisches Mikroskop auf Basis von erweiterter Realität mit optischer Kohärenztomografie (OCT) nach Anspruch 6, wobei die Anzahl der in Schritt S2 benötigten Referenzpunkte n beträgt und n ≥ 6 ist.

## Revendications

1. Système d'imagerie de microscope chirurgical basé sur la réalité augmentée de tomographie en cohérence optique (OCT) , comprenant :
une unité de microscope chirurgical (7), configurée pour acquérir une image microscopique bidimensionnelle d'une région chirurgicale (1) ;
une unité OCT (3), configurée pour acquérir une image tridimensionnelle OCT de la région chirurgicale (1) ;
une unité de contrôle de traitement (8), configurée pour acquérir l'image microscopique bidimensionnelle et l'image tridimensionnelle OCT de la région chirurgicale (1), et une image obtenue en fusionnant l'image microscopique bidimensionnelle et l'image tridimensionnelle OCT de la région chirurgicale (1) ;
une unité d'affichage (9), configurée pour produire et afficher un résultat de l'unité de contrôle de traitement (8) afin d'effectuer la navigation pour la chirurgie ; et
une source lumineuse de guidage (4), qui peut être captée par l'unité de microscope chirurgical (7) et configurée pour projeter dans la région chirurgicale (1) un point lumineux de guidage synchronisé avec une source lumineuse de balayage OCT (301) de l'unité OCT (3) ;
l'unité OCT (3) comprend la source lumineuse de balayage OCT (301), un premier coupleur (302), un multiplexeur à division de longueur d'onde (303), un premier collimateur (304), un scanner galvanométrique bidimensionnel (305), un deuxième collimateur (306), un réflecteur (307), un troisième collimateur (308), un deuxième coupleur (309) et un détecteur d'équilibre (310) ;
un faisceau de balayage OCT émis par la source lumineuse de balayage OCT (301) est divisé en deux chemins lumineux via le premier coupleur (302) : un chemin lumineux est la lumière d'échantillonnage, et l'autre chemin est la lumière de référence ;
la lumière de guidage émise par la source lumineuse de guidage (4) et la lumière d'échantillonnage convergent via le multiplexeur à division de longueur d'onde (303), puis traversent ensemble le premier collimateur (304) pour devenir incidentes avec le scanner galvanométrique bidimensionnel (305) pour être déviées, puis sont focalisées dans la région chirurgicale (1) par une lentille d'objectif (2) après avoir été réfléchies par un miroir dichroïque (501) ;
la lumière d'échantillonnage et une partie de la lumière de guidage réfléchies par la région chirurgicale (1) reviennent le long d'un trajet d'origine après avoir été réfléchies par le miroir dichroïque (501), et atteignent une extrémité du deuxième coupleur (309) après avoir traversé le premier coupleur (302) ; l'autre portion de lumière de guidage réfléchie par la région chirurgicale (1) passe à travers le miroir dichroïque (501) après avoir traversé la lentille d'objectif (2), passe par une unité de zoom optique (6), puis passe respectivement par une lentille d'imagerie gauche (701) et une lentille d'imagerie droite (702) pour entrer respectivement dans une caméra gauche (703) et une caméra droite (704) ;
la lumière de référence émergente après avoir traversé le premier coupleur (302) passe séquentiellement par le deuxième collimateur (306), le réflecteur (307) et le troisième collimateur (308) pour atteindre ladite extrémité du deuxième coupleur (309), et entre dans le deuxième coupleur (309) avec la lumière d'échantillonnage et la portion de lumière de guidage qui ont été réfléchies par la région chirurgicale (1) et ont atteint cette extrémité du deuxième coupleur (309), et la lumière de référence subit une interférence avec la lumière d'échantillonnage et la partie de la lumière de guidage avant d'être reçue par le détecteur d'équilibre (310), et enfin, un résultat de détection est envoyé à l'unité de contrôle de traitement (8) afin de réaliser l'imagerie tridimensionnelle OCT ;
une fois qu'un faisceau de lumière émis par une unité de lumière chirurgicale (10) irradie la région chirurgicale (1), la lumière d'éclairage et l'autre partie de la lumière de guidage réfléchie par la région chirurgicale (1) sont transmises à travers le miroir dichroïque (501), puis passent par l'unité de zoom optique (6) et entrent ensuite dans un module d'imagerie microscopique gauche et un module microscopique droit, et enfin, un signal d'imagerie est envoyé à l'unité de contrôle de traitement (8) afin de réaliser une imagerie microscopique bidimensionnelle de la région chirurgicale (1) ; et
l'unité de contrôle de traitement (8) est configurée pour effectuer l'enregistrement et la fusion sur l'image microscopique bidimensionnelle et l'image tridimensionnelle OCT de la région chirurgicale (1), et une image fusionnée est affichée et produite par l'unité d'affichage (9) afin d'effectuer la navigation pour la chirurgie.

2. Système d'imagerie de microscope chirurgical basé sur la réalité augmentée OCT selon la revendication 1, comprenant en outre une unité de lumière chirurgicale (10), une lentille d'objectif (2), une unité de séparation de la lumière (5) et une unité de zoom optique (6) disposées séquentiellement le long d'un chemin optique d'imagerie de l'unité de microscope chirurgical (7), dans lequel
l'unité de lumière chirurgicale (10) est configurée pour fournir de la lumière à la région chirurgicale (1), et la lumière réfléchie par la région chirurgicale (1) entre dans l'unité de microscope chirurgical (7) après être passée séquentiellement par la lentille d'objectif (2), l'unité de séparation de lumière (5) et l'unité de zoom optique (6) afin de réaliser une imagerie microscopique bidimensionnelle de la région chirurgicale (1) ;
la lumière émise par la source lumineuse de guidage (4) et la source lumineuse de balayage OCT (301) de l'unité OCT (3) atteint la région chirurgicale (1) après être passée séquentiellement par l'unité de séparation de la lumière (5) et la lentille d'objectif (2), et la lumière de balayage OCT réfléchie par la région chirurgicale (1) revient vers l'unité OCT (3) pour réaliser l'imagerie tridimensionnelle OCT ; et après que la lumière de guidage réfléchie par la région chirurgicale (1) traverse l'unité de séparation de la lumière (5), une partie de la lumière de guidage entre dans l'unité OCT (3), tandis que l'autre partie de la lumière de guidage entre dans l'unité de microscope chirurgical (7).

3. Système d'imagerie de microscope chirurgical basé sur la réalité augmentée OCT selon la revendication 2, dans lequel l'unité de microscopie chirurgicale (7) comprend des lentilles d'imagerie et des caméras, les lentilles d'imagerie comprennent une lentille d'imagerie gauche (701) et une lentille d'imagerie droite (702), et les caméras comprennent une caméra gauche (703) et une caméra droite (704), dans lequel la lentille d'imagerie gauche (701) et la caméra gauche (703) constituent respectivement un module d'imagerie microscopique gauche, et la lentille d'imagerie droite (702) et la caméra droite (704) constituent en conséquence un module d'imagerie microscopique droit.

4. Système d'imagerie de microscope chirurgical basé sur la réalité augmentée OCT selon la revendication 3, dans lequel l'unité de séparation de la lumière (5) est un miroir dichroïque (501) qui effectue la réflexion totale sur la lumière de l'unité OCT (3), effectue la semi-transmission et la semi-réflexion sur la lumière de la source lumineuse de guidage (4), et effectue la transmission totale sur la lumière de l'unité de lumière chirurgicale (10).

5. Système d'imagerie de microscope chirurgical basé sur la réalité augmentée OCT selon la revendication 1, dans lequel l'unité d'affichage (9) est un écran à lumière polarisée avec un effet visuel stéréoscopique, et est configuré pour produire respectivement une image obtenue en fusionnant l'image microscopique bidimensionnelle du module d'imagerie microscopique gauche et l'image tridimensionnelle OCT et une image obtenue en fusionnant l'image microscopique bidimensionnelle du module d'imagerie microscopique de droite et l'image tridimensionnelle OCT.

6. Procédé d'imagerie de microscope chirurgical basé sur la réalité augmentée OCT, utilisant le système selon l'une des revendications 1 à 5 pour réaliser l'imagerie, comprenant les étapes suivantes :
S1 : ajuster l'intensité de sortie et des positions de mise au point d'une unité de lumière chirurgicale (10) et d'une source lumineuse de guidage (4) pour permettre aux caméras d'une unité de microscope chirurgical (7) d'observer clairement une zone chirurgicale (1) et un point lumineux de guidage, et acquérir une image microscopique de la région chirurgicale (1) ;
S2 : établir un système de coordonnées cartésiennes bidimensionnel *Ox₀y₀* au microscope en prenant un plan bidimensionnel de l'image microscopique acquise par les caméras comme les axes x *et y* et en prenant le coin supérieur gauche de l'image microscopique comme origine, en obtenant les coordonnées du point lumineux de guidage dans le système de coordonnées du microscope selon la position du point lumineux de guidage dans l'image, et en utilisant les coordonnées obtenues comme point de référence ; et le changement, dans une région de balayage tridimensionnelle OCT, d'un angle de déviation d'un scanner galvanométrique bidimensionnel (305), en acquérant les coordonnées d'une série de points de référence différents à marquer comme {A₁, A₂ ... Aₙ} ;
S3 : établir un système de coordonnées cartésiennes tridimensionnel *Ox₀y₀z₀,* appelé système de coordonnées OCT, en prenant une pluralité de données continues de tranche OCT à des positions adjacentes comme données volumiques, en prenant une direction de balayage en profondeur OCT comme axe z et en prenant les directions de balayage du scanner galvanométrique bidimensionnel (305) comme axes x et y ; en réalisant un balayage OCT tridimensionnel primaire sur une région d'imagerie, où, en raison du fait qu'un angle de déviation du scanner correspondant à la position de projection de la lumière de guidage dans l'étape S2 est connu, les valeurs de coordonnées *x₁* et *y₁,* correspondant à la position du point lumineux de guidage de l'étape S2, dans le système de coordonnées OCT, sont également connues pour trouver une frontière où se trouve le point lumineux de guidage selon une structure OCT, acquérant ainsi des valeurs de coordonnées zi du point lumineux de guidage de l'étape S2 dans le système de coordonnées OCT, et enfin, obtenant les coordonnées *{B₁, B₂ ... Bₙ}* dans le système de coordonnées OCT correspondant aux points de référence *{A₁, A₂ ... Aₙ}* dans le système de coordonnées cartésiennes bidimensionnel *Ox₀y₀ ;*
S4 : effectuer l'ajustement sur *{A₁, A₂ ... Aₙ}* et *{B₁, B₂ ... Bₙ}* pour obtenir une relation de transformation $\left[\begin{matrix}{x}_{0} \\ {y}_{0} \\ 1\end{matrix}\right] = {KT}_{w} \left[\begin{matrix}{x}_{1} \\ {y}_{1} \\ {z}_{1} \\ 1\end{matrix}\right] = P \left[\begin{matrix}{x}_{1} \\ {y}_{1} \\ {z}_{1} \\ 1\end{matrix}\right]$ du système de coordonnées OCT vers le système de coordonnées cartésiennes bidimensionnelles du microscope, qui est une matrice d'homographie correspondant à la transformation de coordonnées, calibrant les caméras pour obtenir les paramètres internes K des caméras, et effectuant une opération matricielle pour obtenir les paramètres externes Tw des caméras ;
S5 : ajuster l'intensité de l'unité de lumière chirurgicale (10), et simultanément commencer à effectuer un balayage tridimensionnel OCT sur la région chirurgicale (1) ;
S6 : obtenir une image OCT reconstruite en trois dimensions enregistrée selon les paramètres des caméras obtenus à l'étape S4, et enfin, effectuer la superposition sur l'image reconstruite tridimensionnelle OCT enregistrée et l'image microscopique de la région chirurgicale (1) pour compléter l'affichage de fusion d'images virtuelles et réelles ; et
S7 : répéter l'étape S6, à mesure que le balayage OCT met à jour en continu les données de volume d'entrée, reconstitue toutes les images structurelles bidimensionnelles pour former un modèle de tomographie tridimensionnelle de la région chirurgicale (1), et effectue l'affichage par une unité d'affichage (9) afin de réaliser une augmentation en temps réel de l'image microscopique de la région chirurgicale (1).

7. Procédé d'imagerie de microscope chirurgicale basé sur la réalité augmentée OCT selon la revendication 6, comprenant : établir les systèmes de coordonnées microscopiques correspondant respectivement à la caméra gauche (703) et à la caméra droite (704), puis effectuer respectivement l'enregistrement et la fusion avec une image OCT.

8. Procédé d'imagerie de microscope chirurgicale basé sur la réalité augmentée OCT selon la revendication 6, dans lequel, lorsque la position du point de référence est fixée, l'angle de déviation du scanner galvanométrique bidimensionnel (305) est une valeur lors du balayage tridimensionnel OCT, au lieu d'une valeur aléatoire dans une plage balayable.

9. Procédé d'imagerie de microscope chirurgicale basé sur la réalité augmentée OCT selon la revendication 6, où un nombre de points de référence requis à l'étape S2 est n, et n≥6.
